# EUROPEAN PATENT SPECIFICATION

(11) **EP 2 174 127 B1**
(45) Date of publication and mention of the grant of the patent: **01.08.2012**
(21) Application number: 08827584.7
(22) Date of filing: 19.05.2008
(51) Int. Cl.: G01N 33/00, G01N 27/12

(54) **SMARTCARD FOR CHEMICAL, BIOLOGICAL, RADIATION OR EXPLOSIVES DETECTION**
SMARTCARD FÜR CHEMISCHE, BIOLOGISCHE, STRAHLUNGS- ODER SPRENGSTOFFDETEKTION
DETECTEUR D'AGENTS CHIMIQUES, BIOLOGIQUES, RADIOLOGIQUES ET EXPLOSIFS DOTE D'UNE CARTE A PUCE

(30) Priority: 17.05.2007 US 938677 P
(43) Date of publication of application: 14.04.2010
(73) Proprietor: Cubic Corporation, San Diego, CA 92123 (US)
(72) Inventor: RAVENIS, Joseph VJ, San Diego, California 92120 (US); DEKOZAN, Raymond, La Mesa, California 91941 (US); BONNEAU, Walter C., Jr., Escondido, California 92025 (US); MACKLIN, Jon D., El Cajon, CA 92021 (US); ROBERTS, Bruce D., San Diego, California 92128 (US)
(74) Representative: Price, Nigel John King
(86) International application number: PCT/US2008/064158
(87) International publication number: WO 2009/025894

(56) References cited:
- WO-A-2006/035392
- WO-A-2006/130528
- DE-A1- 4 322 274
- US-A1- 2002 024 450
- US-A1- 2003 143 119
- US-A1- 2005 022 581
- US-A1- 2005 088 299
- US-A1- 2006 290 496
- US-A1- 2007 102 294

## Description

### BACKGROUND OF THE INVENTION

This disclosure relates in general to chemical, biological, radiation and explosive detection and, but not by way of limitation, to smartcard detection.

Detection of trace particles or emanations from compounds which may represent a threat to the public is based on the capture and analysis of the material. Capture may be accomplished through contact (e.g., wipe a surface or contact with a capture surface) or through capture from the atmosphere (e.g., forced air flow such as a "puffer" to dislodge particles from surfaces or through vapor sampling from the atmosphere).

Analysis in most current systems employs ion mobility spectroscopy as the mechanism for detecting items of interest. The detection capture and analysis devices may be installed in the infrastructure being protected such as at the portals for entry or exit, positioned to capture from the persons involved through contact (e.g., touch or swipe) or may be handheld and employed by those protecting the infrastructure. Such devices are common in airports today. The devices typically are slow in the capture and analysis process, frequently require operator participation and require regular cleaning, potentially after each use. In addition, the analysis results are frequently ambiguous, resulting in high false alarm rates.

An emerging class of detection devices relies on the capture of the threat indicating material causing a change in the composition of the material of the device which captures it. The change is then observable or causes a detectable change in the reflective photo luminescence. For example, film tags are used in nuclear facilities to determine if there has been exposure to radiation. Optical scanners may be employed to detect the change in luminescence when the capture material is presented. In cases where the change in the capture material is visible, the holder may dispose of the device before the capture event is recorded.

DE 4322274 discloses the pre-characterising sections of claims 1 and 9.

US 2002/0024450 discloses a data collection apparatus includes a sensing unit for attaching to a structure or live subject for sensing a parameter of the structure or live subject. The sensing unit includes a sensor, a data storage device, and a transmitting device. The data storage device is for storing data from the sensor. The apparatus also includes a control unit that is separable from the sensing unit. The control unit includes a data receiving device and a second data storage device different from said first storage device. The data receiving device is to receive data transmitted from the data sensing unit. The second data storage device is for storing data receiving from the sensing unit. The apparatus also includes a triggering device for modifying the storing of data being stored to the first data storage device or for initiating transmission of data from the sensing unit to the control unit. The triggering device is controlled by a real time change in information about the structure or live subject.

### BRIEF SUMMARY OF THE INVENTION

According to the invention there is provided a detector card for recording exposure to an item of interest according to appended claim 1.

According to the invention there is provided a method for detecting items of interest with a detector card, the method including the steps of appended claim 9.

Further areas of applicability of the present disclosure will become apparent from the detailed description provided hereinafter. It should be understood that the detailed description and specific examples, while indicating various embodiments, are intended for purposes of illustration only and are not intended to necessarily limit the scope of the disclosure.

### BRIEF DESCRIPTION OF THE DRAWINGS

The present disclosure is described in conjunction with the appended figures:
FIG. 1 depicts a block diagram of an embodiment of a detection system in an environment of items of interest;
FIGs. 2A and 2B depict block diagrams of embodiments of a wireless detector circuit;
FIGs. 3A and 3B depict diagrams of embodiments of a detector card;
FIGs. 4A illustrates a flowchart of a process for detecting items of interest with a detector card;
FIGs. 5A, 5B and 5C depict a structural diagram of an embodiment of the detector card having three layers of construction;
FIGs. 6A and 6B depict diagrams of an embodiment of detection and conduction polymer before and after detection; and
FIG. 7 depicts a diagram of an embodiment of a layered polymer configuration fr a sensor.

In the appended figures, similar components and/or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

### DETAILED DESCRIPTION OF THE INVENTION

In one embodiment, a chemiselective or detection polymer captures trace particles or emanations from a specific threat compound. A conductive polymer senses the change in a characteristic of the detection polymer after the capture of the target particle or emanation. The exposure information is transferred to the electronic circuit embedded in the smartcard or token. The electronic circuit wirelessly communicates with a reader in a manner consistent with the present or future reader standards.

In another embodiment, a smartcard is used for access control or security systems operating at 13.56MHz or higher with the ability to detect and report trace CBRE material that has been in contact with the smartcard. When the card communicates with any type of Automatic Fare Collection system such as those found in, public transportation systems, automated parking systems, stadium event ticketing systems or building access systems, the trace detection status of what has come in contact with the smartcard is reported through the infrastructure in order to provide detection, intelligence gathering information, and prevention of terrorist incidents. This information may be used for intelligence collection into a special situational awareness software program or interface into a command and control (C2) or communication, command and control (C3) system.

A smartcard using a polymer sensor technology, such as but not limited to, fluorescent quenching or molecularly imprinted polymer (MIP) technology can register detection of a substance that has come in contact with the card when in an powered or non-powered state. These technologies interact with an additional conductive polymer and/or nanotechnology layer(s). The detection polymer and the conductive polymer or nanotechnology may be amalgamated or conjunctively combined. When the detection polymer is contaminated with item of interest, it interacts with the other polymer materials, and a signal is generated and relayed to a microprocessor or memory cell located in a smartcard. The interaction can be through a chemical, physical, or electronic change. The change signifies that a detection of a target substance or substances has occurred. The detection event triggers changes in an electrical or data characteristic of the smartcard that corresponds to the specific sensors targeted triggering substance. Each card can have one or many detection sensor inputs and can be configurable to accept combinations of any CBRE substances.

The detector card is powered when it is placed within the electromagnetic field generated by a reader, such that detection of trace materials can be reported wirelessly to the reader to deter, prevent or contain the potential threat should it be validated. In addition to being able to detect the item of interest, it may also provide an indication of the volume or strength of trace materials detected.

Detection polymers exist for most explosive and chemical threat agents. Additionally, materials can capture radiation emanations and polymers for biological material. Embodiments of the invention can contain one or multiple polymers for detection (e.g., fluorescent quenching or MIP) and one or multiple polymers and nanotechnologies for conducting signals to the electronic circuitry. These two types of polymers can be amalgamated or conjunctively joined into a card substrate. Carbon nanotubes and other nanotechnology can be used for printed electronic circuits and to interface with the conducting polymer. The application of the polymers, combined in either a mixed or a layered scheme can be applied using multiple methods, as a polymer/nanotech ink, using a spray method, brushing, spin-coating, printing, and/or roller-coating. Ink jet printing technology can be used, for example, to spray apply the polymer(s) and form nanotech circuits on or in the surface or substrate materials. According to the invention the smartcard transfers the detection event data to the reader employing standard smartcard communication methods.

A smartcard is used by individuals in a number of applications that are germane to this invention. A smartcard is of a contactless type being read wirelessly at a distance. The detector card may be a token or credential (e.g., badge, ID card, license, etc.), a bank card (e.g., credit, debit, stored value, etc.), or a preferred customer or member card or a prepaid card for other economic applications (e.g., transit system fares, NFC enabled cellular phone, prepaid gift cards, etc.). The detector card or media fits within the palm of a human hand, but other embodiments could be less than 64.5, 51.6, 38.7, 32.3, 25.8, 19.4, 12.9,6.5 cm² (10, 8, 6, 5, 4, 3, 2, or 1 in²) and thinner than 10, 8, 6, 5, 4, 3, 2, or 1 mm.

Currently available are a class of conductive polymers that have conductivity levels between those of semiconductors and metals. Until recently, these conductive polymers did not have sufficient conductive properties to be utilized in manner of this invention. Conductive polymers, such as but not limited to, the highly conductive Clevios™ series available HS Starck™ that provides the base material for an electrical conversion for the detection polymer (e.g., MIP).

The combination of the detection polymer with a conductive polymer provides the basic component used in a sensor that can detect and have an electrical property change that can be electronically relayed. The combination of the two polymers is performed as an amalgamated polymer or a conjunctively combined polymer. The currently commercially available conductive polymers have a conductivity rating up to 1500 ohms/cm² that allows for an electromagnetic field to provide enough induced power to quantify an electrical characteristic change in the detection polymer. This change will occur when the detection polymer moves from an uncontaminated to a contaminated state.

Nanotechnology techniques, such as but not limited to, carbon nanotubes, can be used to form the circuit that can discriminate the signals generated from the contaminated detection polymers. The electrical signals can be developed through changes in inductive coupling, capacitive coupling, magnetic coupling or resistivity.

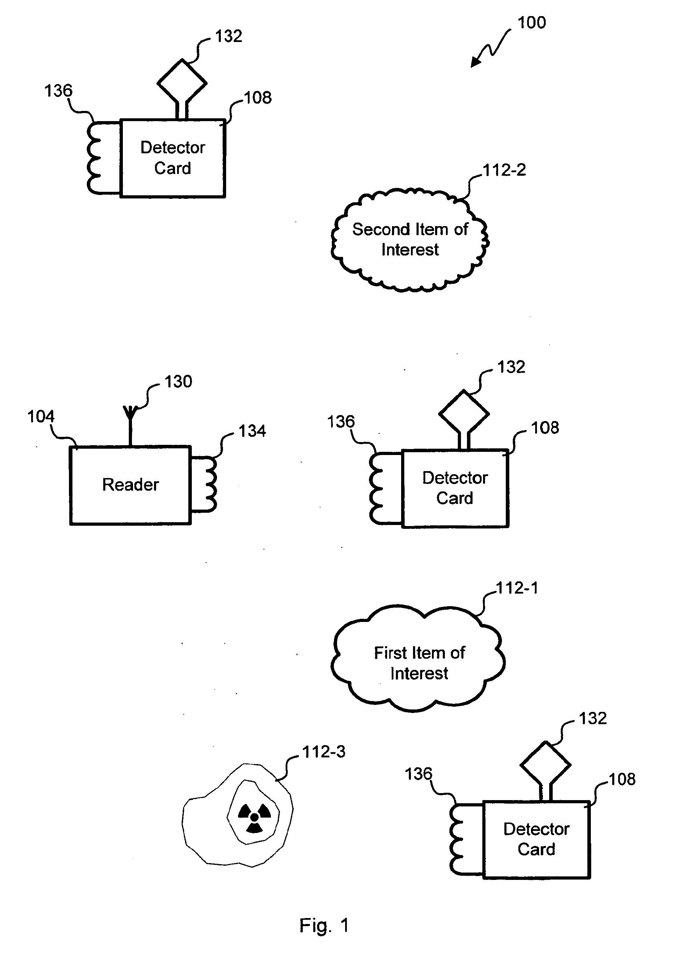

Referring initially to **FIG. 1**, a block diagram of an embodiment of a detection system 100 in an environment of items of interest 112 is shown. This embodiment has several detector cards or media 108 that each communicate with an antenna 132 and receive power inductively through a coil 136. The detector media 108 are exposed to various items of interest 112 as they travel with an associated user. The detector media 108 are capable of sensing exposure to one or more items of interest, even when the coil 136 is not receiving power. Any exposure can be communicated as detection information to a reader or reader 104.

The detector cards or media 108 communicate with readers using wireless radio frequencies (RF), for example, 13.56 MHz or higher frequency signals could be used. The detector card 108 or token could be used as a smartcard for other purposes. For example, the detector card 108 could be used for access into a system such as, public transportation systems, automated parking systems, stadium event ticketing systems or building access systems. Additionally, the detector card 108 could be part of a phone or credit or debit card. The detector media 108 need not be in card form and can be any type of RF identification (RFID).

At any time, a reader 104 can wirelessly power a detection media 108 and communicate with the detector media 108 within communication range. Readers 104 could be handheld or fixed, but can read any detection information in nearby detector media 108 from a distance. The reader uses a coil 134 to send power to the detector media 108. An antenna 130 of the reader 104 is used to communicate with the detector media 108.

Various items of interest 112 can be sensed by the detector card 108. The items of interest could be a chemical, a biologic compound, radiation, and/or an explosive (CBRE), for example. As the detector card 108 is carried by the user, any exposure to these items of interest 112 can be sensed and that information retained in some manner until a reader 104 reads the information. The item of interest may be directly transferred by contact or indirectly without any contact.

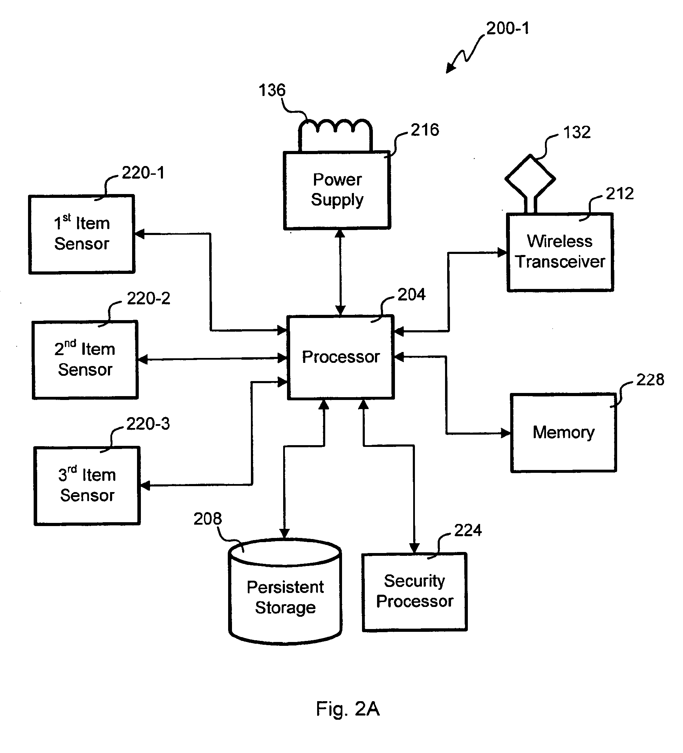

With reference to **FIG. 2A**, a block diagram of an embodiment of a wireless detector circuit 200-1 is shown. The detector circuit 200-1 is embedded in a detector card 108 . A processor 204 or microcontroller runs software using the memory 228. The software can be held in the persistent storage 208 such as flash, ROM or some other non-volatile memory. The persistent storage 208 can be used to store identifiers for the wireless detector circuit 200-1 and sensor readings. Various amounts of historical sensor readings can also be stored in the persistent storage 208.

This detector card 108 is used as a smartcard. A security processor 224 can be used for authentication, authorization or secure storage of information. Other embodiments could be used for no more than sensing items of interest without the other smartcard functionality.

A wireless transceiver 212 allows bi-directional communication with the wireless detector circuit 200. The antenna 132 is used for this communication. Other embodiments could have multiple transceivers and antenna tuned to other frequencies.

A power supply 216 allows intermittent energy supply to the wireless detector circuit 200. When in range with a reader 104, energy is coupled to the coil 136 and converted into appropriate voltages by the power supply 216. The wireless detector circuit 200 becomes fully functional when properly energized by the reader 104.

The passive sensors 220 do not require power to record exposure to items of interest. For example, fluorescent quenching polymers or molecularly imprinted polymer (MIP) technology can report detection of a substance that has come in contact with the item sensor 220 when the wireless detector circuit 200 is in an powered or non-powered state. The item sensor 220 can read a chemical, physical, or electronic change in the MIP. The change signifies that a detection of a target substance or substances has occurred. Each item sensor 220 can be configured to be sensitive to one or more compounds or conditions.

When the wireless detector circuit 200 is next powered, the exposure of the detection polymer can be recorded in the persistent storage 208 as exposure information. The value of the exposure information can be a value indicative of the amount of exposure experienced. The characteristics of the detection polymer can be such that the resistance (or some other electrically readable characteristic) changes as a function of exposure.

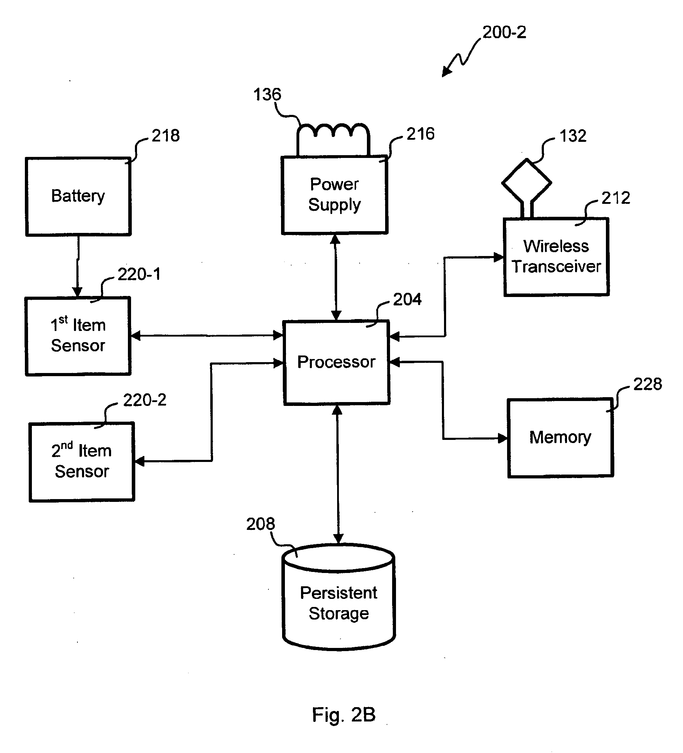

Referring next to **FIG. 2B**, a block diagram of another embodiment of a wireless detector circuit 200-2 is shown. This embodiment doesn't include the security processor 224. The wireless detector circuit 200 has two item sensors 220. The second item sensor 220-2 reacts with one or more items of interests without requiring power. The first item sensor 220-1 uses battery power to detect one or more items of interest. The battery 218 can be used by the first item sensor 220-1 to detect and/or hold the exposure information.

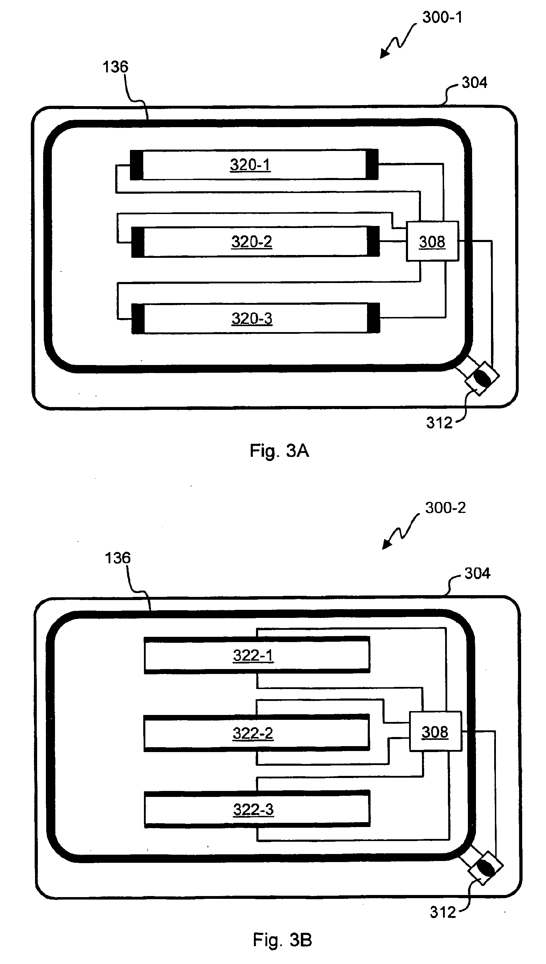

With reference to **FIG. 3A**, a cross-sectional diagram of an embodiment of a detector card 108 is shown. Various items are embedded into a media 304, which could be made from plastic, for example. This embodiment has three item sensors 320 that change resistivity with a detection polymer. The resistance is measured by sensor electronics 308 and reported back to an embedded chip or integrated circuit (IC) 312 for recording the exposure information. The embedded chip could include memory 228, a processor 204, a security processor 224, persistent storage 208, a wireless transceiver 212, and a power supply 216.

Referring next to **FIG. 3B**, a cross-sectional diagram of another embodiment of a detector card 108 is shown. This embodiment has three item sensors 322 that are configured differently from the embodiment of FIG. 3A to measure changes in capacitance. Various embodiments of the item sensors 322 could change inductance, magnetism, resistance, capacitance, opaqueness, etc. Generally, the detection polymer changes the electrical characteristics of item sensors 322 as a function of exposure to an item of interest 112. The detection polymer binds with the item of interest during exposure.

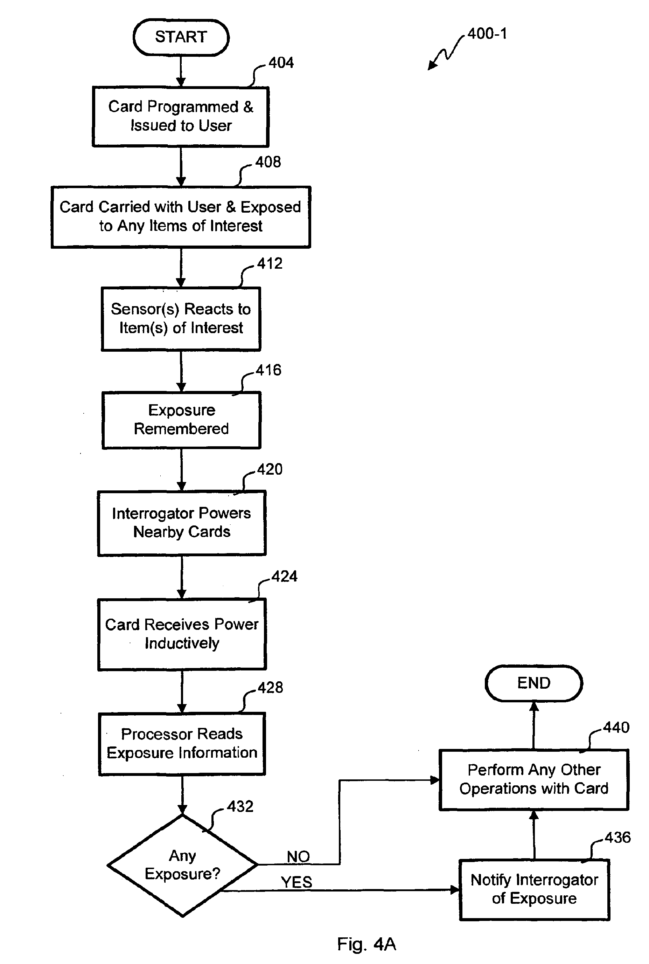

With reference to **FIG. 4A**, a flowchart of an embodiment of a process 400-1 for detecting items of interest with a detector card 108 is shown. The depicted portion of the process 400-1 begins in block 404 where the detector card is issued to a user along with any programming. For example, the detector card 108 could be written with user information, applications, user preferences, serial numbers, and/or other information. The user carries around the detector card 108 where it potentially is exposed to items of interest in block 408.

In block 412, the item sensor 220 reacts to exposure to the relevant item(s) of interest. Any exposure is remembered as exposure information in block 416. The exposure information maybe stored in the sensor material using a detection polymer, for example, or some other material sensitive to the item(s) of interest.

At some point, the detector card 108 comes in contact with a reader 104 in block 420 that powers the coil 136 of the detector card 108 to power up the detector circuit 200 in block 424. The processor 204 reads one or more item sensors 220 in block 428. The detection polymer remembers the exposure that can be read at any time as exposure information. The exposure information could be a range of values.

Where there is exposure detected in block 432, the reader 104 is sent the exposure information wirelessly in block 436. Processing continues from block 436 to block 440 where any other operations are performed with the detector card 108. Where exposure hasn't been detected, processing goes from block 432 to block 440 to perform any other operations with the detector card 108 that the reader 104 might perform.

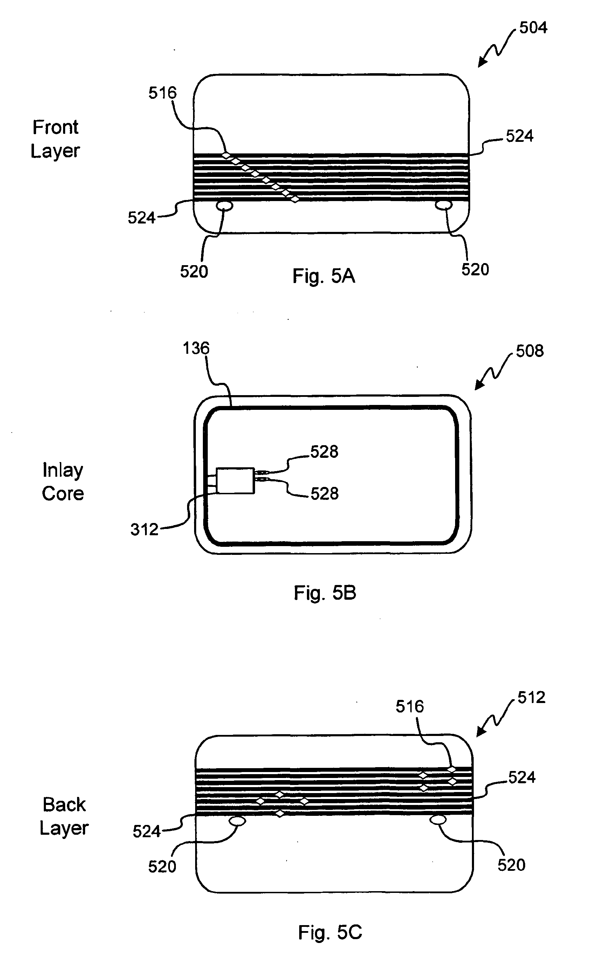

Referring next to **FIGS. 5A**, **5B** and **5C**, a structural diagram of an embodiment of the detector card having three layers of construction is shown. The configuration can be changed dependent upon the requirements of the media that the polymers will be embedded and the ergonomics of the device to enhance detection from the expected handling of the detector card. The front and back layers 504, 512 are made of a plastic, plasticized, polyvinyl or paper material in which various layers of amalgamated, aggregated, or conjunctively combined conductive polymers 520 and detection polymers 524 are applied in an interlaced or checkered pattern, for example. This pattern can vary with ergonomic requirements.

Each of these detection polymer stripes 524 are integrated with a sensor 516 that transfers the conductivity change in value to the inner layer or inlay core 508 using sensor connection points 528. The inlay core 508 processes the sensor information and acts as a transmitter to either the integrated circuit (IC) 312 or printed circuit (PC) contact point, which could include the use of the existing antenna inputs. A method of detuning the antenna may be implemented as well when the detection polymer changes in characteristics due to a threat substance exposure. The change in characteristics of the detection polymer may be inductive, voltage, resistance, and conductive and/or magnetic in nature.

The IC or PC 312 will evaluate the change in input characteristics during and when a detection change occurs, or simply reacts to the change in antenna Q factor and or both to trigger the IC or PC 312 to take the appropriate action to signal that a detection occurrence took place. In turn, the smartcard reader (or PCD) 104 interrupts this change in signal detection and transmits or sends the appropriate information to the host processing system for a valid response.

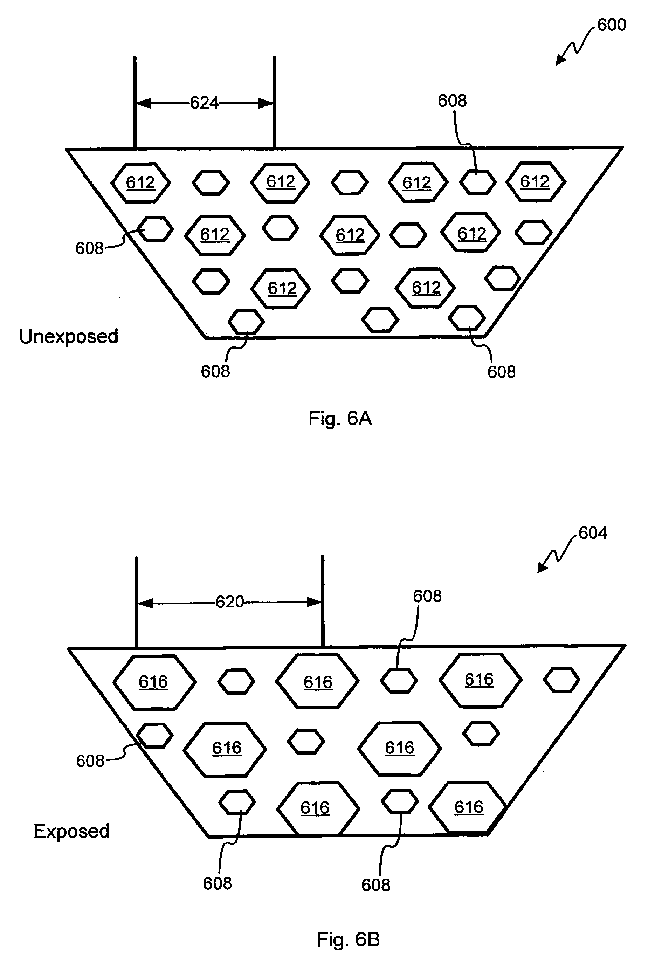

Referring next to **FIGs. 6A** and **6B**, a diagram of an embodiment of detection and conduction polymer is shown both before 600 and after detection 604. In this embodiment, the detection and conduction polymers are mixed or interspersed. The polymer materials being applied to the detector card include two types of base polymers that include a conductive polymer 608 and the other a chemical, biological, radiological or explosives detection sensitive polymer 612, 616. The unexposed detection polymer 612 is shown in FIG. 6A and the exposed detection polymer 616 is shown in FIG. 6B. In this example, the detection polymer 612, 616 changes the state of the conductive polymer 608 by decreasing or increasing the conductivity of the conductive polymer 608.

In this embodiment, the spacing relationship between the conductive polymer 608 and the detection polymer 612 is a known distance yielding a known value of conductivity measured in ohms/cm². The distance between the conductive polymer particles or monocles will increase resulting in an ohmic value increase, or the conductivity will decrease when the detection polymer 612, 616 is subjected with a threat substance in which the detection polymer 612, 616 is designed to respond. The smaller distance 624 is shown in FIG. 6A and the larger distance 620 is shown in FIG. 6B.

As the detector card 108 is carried and handled by the user any item of interest (CBRE particulates or vapors) will cause the unseen reaction in the detection polymer 612, 616 and the event will either be stored or set for reading when external power is applied to the detector card 108. In the embodiment of a detector card that contains a power source, the event can be detected and read once the detection polymer 612, 616 has provided a large enough change of electrical properties such that it can be measured by the microprocessor or IC 312. There are various embodiments to demonstrate how the polymers can be arranged and methods of deposition onto a card, ticket or other surface.

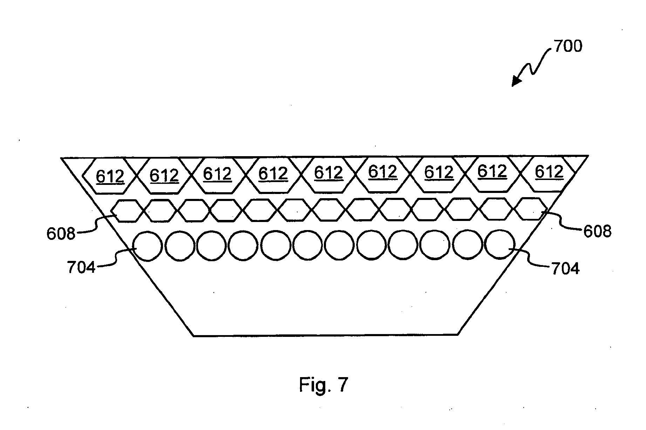

Referring next to **FIG. 7**, an embodiment of a layered polymer configuration for a sensor is shown. This embodiment demonstrates how the polymers can be combined conjunctively, in a layered manner. In this embodiment, the detection polymer is not mixed with the conductive layer as in the embodiments of FIGs. 6A and 6B, but is applied after the conductive layer is dried or cured.

When the detection polymer 612 is exposed to the particulates or vapors of interest, a change in the detection polymer 612 allows the conductive polymer 608 and nanotechnology layer 704 to read that change. This reading is based on changes of resistance, capacitance, magnetic coupling or inductive coupling that can occur when the detection polymer 612 is contaminated with a specific item of interest such as a particular explosive material component, a chemical material component, or a radiation component, or biological contamination.

This signal is stored and forwarded into an IC and/or hosting system. This process can occur during the initial communication phase with a smartcard reader.

The smartcard reader can be a portable unit or one that is mounted in a permanent or semi-permanent location. In this embodiment, the detection polymer(s) 612 can be more than one selectively sensitive polymer applied in a bar code type of arrangement that would allow for the detection of multiple specific items of interest. The electrical change sensor would be connected to the multiple areas of each specific polymer to allow for the identification of the specific item of interest that was detected.

Detection polymers are polymers that are currently available to detect a particular item of interest. They could include vapor or particulate sensing polymers, florescent quenching polymers, and/or Molecularly Imprinted Polymers (MIP). Current classifications of sensing would include Chemical, Biological, Radiation and Explosive (CBRE). The molecular formula and the electrical properties for each classification of substances vary, as well as the formulations for each subclass. For example; the molecular formula for a MIP polymer that detects TNT will vary from the molecular formula for the MIP polymer that detects RDX. These differing formulas cause the electrical properties, conductance or resistance, of each formulation to typically be different.

Each detection polymer type is matched with a specific formulation of conductive polymer and/or nanotechnology particulates, such that a electrical signature can be read once it is exposed to the specific analyte that causes the positive chemical reaction in the chemi-selective detection polymer. The value of the exposure information can be a value indicative of the amount of exposure experienced. The characteristics of the MIP can be such that the resistance (or some other electrically readable characteristic) changes as a function of exposure.

While the principles of the disclosure have been described above in connection with specific apparatuses and methods, it is to be clearly understood that this description is made only by way of example and not as limitation on the scope of the disclosure.

## Claims

1. A smartcard detector card (108) for recording exposure to an item of interest, the smartcard detector card comprising:
a media (304);
an electronic circuit (200) embedded in the media, wherein the electronic circuit is wirelessly readable; and
a sensor (220) that reacts when exposed to the item of interest without requiring the smartcard detector card to be in a powered state;
**characterised in that** the detector further comprises:
an antenna (132) configured to send exposure information to a reader (104) by employing standard smartcard communication methods, wherein the antenna is embedded in the detector card;
a power source (216) that inductively couples energy into the smartcard detector card (108); and
wherein the electronic circuit (200) can detect if the sensor (220) has reacted only when powered by the power source(216).

2. The smartcard detector card of claim 1, wherein the sensor comprises either:
molecularly imprinted polymer(s); or
fluorescent quenching polymer(s); or
a plurality of layers conjunctively formed with detection polymer(s) and conductive polymer(s).

3. The smartcard detector card of claim 1, wherein the sensor includes material that is either:
formed with a detection polymer(s) and a conducting polymer(s); or
applied in an ink form to the media.

4. The smartcard detector card of claim 1, wherein the sensor includes a material that comprises either:
a plurality of layers of an amalgamated polymer formed with a detection polymer(s) and a conductive polymer(s); or
a plurality of layers conjunctively formed with a detection polymer(s), a conductive polymer and nanotechnology; or
a plurality of layers of an amalgamated polymer formed with a detection polymer(s), a conductive polymer(s) and nanotechnology combination.

5. The smartcard detector card of claim 1, further comprising :
a second sensor (220) that reacts when exposed to a second item of interest, wherein the electronic circuit can detect if the second sensor has reacted; and
a battery (218) that aids the sensor in sensing the item of interest.

6. The smartcard detector card of claim 1, wherein the sensor includes a material, wherein either:
the material is reactive to a chemical, a biologic compound, radiation, and/or an explosive; or
a state of the material after reaction is not visible to the unaided human eye.

7. The smartcard detector card of claim 1, wherein the power source comprises either:
a coil (213) that inductively couples power to the electronic circuit.

8. The smartcard detection card media of claim 1, wherein:
the sensor includes a polymer that transitions to different state when exposed to the item of interest.

9. A method for detecting items of interest with a smartcard detector card (108), the method including steps of:
sensing, with a sensor (220) of the smartcard detector card, exposure to an item of interest whilst the smartcard detector card may be in a non-powered state, to create exposure information;
storing the exposure information;
**characterised by**:
powering an electronic circuit (200) of the detector card with a power source (216) that inductively couples energy into the smartcard detector card,
wherein the electronic circuit (220), when powered, can detect a past exposure to the item of interest; and
the method further comprises wirelessly providing the exposure information from the detector card by employing standard smartcard communication method.

10. The method of claim 9, wherein the storing step comprises a step of storing with either:
a reactive polymer; or
electronic memory.

## Patentansprüche

1. Smartcard-Detektorkarte (108) zum Aufzeichnen des Ausgesetztseins gegenüber einem Probengegenstand, wobei die Smartcard-Detektorkarte umfasst:
ein Medium (304);
einen elektronischen Schaltkreis (200), der in dem Medium eingebettet ist, wobei der elektronische Schaltkreis drahtlos auslesbar ist; und
einen Sensor (220), der reagiert, wenn er dem Probengegenstand ausgesetzt ist, ohne dass benötigt wird, dass die Smartcard-Detektorkarte in einem mit Energie versorgten Zustand ist;
**dadurch gekennzeichnet, dass** der Detektor weiterhin umfasst:
eine Antenne (132), die konfiguriert ist, Ausgesetztsein-Information an ein Lesegerät (104) durch Anwendung von herkömmlichen Smartcard-Kommunikationsverfahren zu senden, wobei die Antenne in die Detektorkarte eingebettet ist;
eine Energiequelle (216), die induktiv Energie in die Smartcard-Detektorkarte (108) einkoppelt; und
wobei der elektronische Schaltkreis (200) nur dann detektieren kann, ob der Sensor (220) reagiert hat, wenn er durch die Energiequelle (216) mit Energie versorgt ist.

2. Smartcard-Detektorkarte nach Anspruch 1, wobei der Sensor entweder umfasst:
ein oder mehrere molekular aufgeprägte Polymer(e); oder
ein oder mehrere fluoreszierende abgeschreckte Polymer(e); oder
eine Vielzahl von verbindend ausgebildeten Lagen mit einem oder mehreren Detektionspolymer(en) und einem oder mehreren leitenden Polymer (en) .

3. Smartcard-Detektorkarte nach Anspruch 1, wobei der Sensor ein Material beinhaltet, das entweder:
mit einem oder mehreren Detektionspolymer(en) und einem oder mehreren Leitungspolymer(en) ausgebildet ist; oder
in einer Tintenform auf das Medium angewendet ist.

4. Smartcard-Detektorkarte nach Anspruch 1, wobei der Sensor ein Material beinhaltet, das entweder umfasst:
eine Vielzahl von Lagen eines vereinigten Polymers, das mit einem oder mehreren Detektionspolymer(en) und einem oder mehreren Leitungspolymer(en) ausgebildet ist; oder
einer Vielzahl von Lagen, die verbindend ausgebildet sind mit einem oder mehreren Detektionspolymer(en), einem Leitungspolymer und Nanotechnologie; oder
einer Vielzahl von Lagen eines vereinigten Polymers, das mit einem oder mehreren Detektionspolymer(en), einem oder mehreren Leitungspolymer(en) und Nanotechnologiekombination ausgebildet ist.

5. Smartcard-Detektorkarte nach Anspruch 1, weiterhin umfassend:
einen zweiten Sensor (220), der reagiert, wenn er einem zweiten Probengegenstand ausgesetzt ist, wobei der elektronische Schaltkreis detektieren kann, ob der zweite Sensor reagiert hat; und
eine Batterie (218), die den Sensor beim Erkennen des Probengegenstands hilft.

6. Smartcard-Detektorkarte nach Anspruch 1, wobei der Sensor ein Material beinhaltet, wobei entweder:
das Material reaktiv auf eine chemische, eine biologische Komponente, Strahlung, und/oder einen Sprengstoff ist; oder ein Zustand des Materials nach Reaktion nicht sichtbar für das nicht unterstützte menschliche Auge ist.

7. Smartcard-Detektorkarte nach Anspruch 1, wobei die Energiequelle entweder umfasst:
eine Windung (213), die induktiv Energie in den elektronischen Schaltkreis einkoppelt.

8. Smartcard-Detektorkartenmedium nach Anspruch 1, wobei:
der Sensor ein Polymer beinhaltet, das in unterschiedliche Zustände übergeht, wenn es dem Probengegenstand ausgesetzt ist.

9. Verfahren zum Detektieren von Probengegenständen mit einer Smartcard-Detektorkarte (108), wobei das Verfahren die Schritte beinhaltet:
Erkennen, mit einem Sensor (220) der Smartcard-Detektorkarte, das Ausgesetztsein gegenüber einem Probengegenstand, während die Smartcard-Detektorkarte in einem nicht mit Energie versorgten Zustand sein kann, um Ausgesetztsein-Information zu erzeugen;
Speichern der Ausgesetztsein-Information;
**gekennzeichnet durch**:
Mit-Energie-Versorgen eines elektronischen Schaltkreises (200) der Detektorkarte mit einer Energiequelle (216), die induktiv Energie in die Smartcard-Detektorkarte einkoppelt, wobei der elektronische Schaltkreis (200), wenn er mit Energie versorgt ist, ein zurückliegendes Ausgesetztsein gegenüber dem Probengegenstand detektieren kann; und
wobei das Verfahren weiterhin das drahtlose Bereitstellen der Ausgesetztsein-Information von der Detektorkarte **durch** Anwenden von herkömmlichen Smartcard-Kommunikationsverfahren umfasst.

10. Verfahren nach Anspruch 9, wobei der Speicherschritt den Schritt des Speicherns mit entweder:
einem reaktiven Polymer; oder
einem elektronischen Datenspeicher umfasst.

## Revendications

1. Carte de détecteur de carte à puce (108), destinée à enregistrer l'exposition à un élément intéressant, la carte de détecteur de carte à puce comprenant :
un support (304) ;
un circuit électronique (200), logé dans le support, dans lequel le circuit électronique est lisible sans fil et
un détecteur (220), qui réagit lorsqu'il est exposé à l'élément intéressant, sans exiger que la carte de détecteur de carte à puce soit dans un état actionné ;
**caractérisée en ce que** le détecteur comprend, en outre :
une antenne (132), configurée pour envoyer les informations sur l'exposition à un lecteur (104), en employant des procédés normalisés de communication par carte à puce, dans lesquels l'antenne est logée dans la carte du détecteur ;
un bloc d'alimentation (216), qui couple de manière inductive l'énergie dans la carte de détecteur de carte à puce (108) et
dans lequel le circuit électronique (200) peut détecter si le détecteur (220) a réagi uniquement lorsqu'il est actionné par le bloc d'alimentation (216).

2. Carte de détecteur de carte à puce selon la revendication 1, dans laquelle le détecteur comprend :
soit un ou des polymère(s), imprimé(s) de manière moléculaire,
soit un ou des polymère(s) de désactivation fluorescent(s),
soit une pluralité de couches, formées de manière conjonctive avec le(s) polymère(s) de détection et le(s) polymère(s) conducteur(s).

3. Carte de détecteur de carte à puce selon la revendication 1, dans laquelle le détecteur comporte un matériau qui est :
soit formé avec un ou des polymère(s) de détection et un ou des polymère(s) conducteur(s),
soit appliqué dans une forme d'encre sur le support.

4. Carte de détecteur de carte à puce selon la revendication 1, dans laquelle le détecteur comporte un matériau qui comprend :
soit une pluralité de couches d'un polymère amalgamé, formé avec un ou des polymère(s) de détection et un ou des polymère(s) conducteur(s),
soit une pluralité de couches, formées de manière conjonctive avec un ou des polymère(s) de détection, un polymère conducteur et de la nanotechnologie,
soit une pluralité de couches d'un polymère amalgamé, formé avec un ou des polymère(s) de détection, un ou des polymère(s) conducteur(s) et une combinaison de nanotechnologie.

5. Carte de détecteur de carte à puce selon la revendication 1, comprenant, en outre :
un deuxième détecteur (220), qui réagit lorsqu'il est exposé à un deuxième élément intéressant, dans lequel le circuit électronique peut détecter si le deuxième détecteur a réagi et
une batterie (218), qui aide le détecteur à détecter l'élément intéressant.

6. Carte de détecteur de carte à puce selon la revendication 1, dans laquelle le détecteur comporte un matériau, dans lequel :
soit le matériau est réactif à une substance chimique, un composé biologique, la radiation et / ou une matière explosive,
soit un état du matériau après réaction n'est pas visible à l'oeil nu.

7. Carte de détecteur de carte à puce selon la revendication 1, dans laquelle le bloc d'alimentation comprend une bobine (213), qui couple, de manière inductive, l'alimentation au circuit électronique.

8. Support de carte de détection de carte à puce selon la revendication 1, dans lequel le détecteur comporte un polymère, qui passe à un état différent lorsqu'il est exposé à l'élément intéressant.

9. Procédé, destiné à détecter des éléments intéressants avec une carte de détecteur de carte à puce (108), le procédé comportant des étapes, consistant à :
détecter, avec un détecteur (220) de la carte de détecteur de carte à puce, l'exposition à un élément intéressant, tandis que la carte de détecteur de carte à puce peut être dans un état non actionné, pour créer des informations sur l'exposition ;
stocker les informations sur l'exposition ;
**caractérisé par** les étapes, consistant à :
alimenter le circuit électronique (200) de la carte de détecteur avec un bloc d'alimentation (216), qui couple, de manière inductive, l'énergie dans la carte de détecteur de carte à puce ;
dans lequel le circuit électronique (200), lorsqu'il est actionné, peut détecter une exposition passée à l'élément intéressant et
le procédé comprend, en outre, sans fil, l'opération, consistant à fournir les informations sur l'exposition de la carte de détecteur en employant un procédé normalisé de communication par carte à puce.

10. Procédé selon la revendication 9, dans lequel l'étape de stockage comprend une étape, consistant à stocker avec :
soit un polymère réactif,
soit une mémoire électronique.
